# EUROPEAN PATENT SPECIFICATION

(11) **EP 1 866 409 B1**
(45) Date of publication and mention of the grant of the patent: **12.10.2011**
(21) Application number: 06739260.5
(22) Date of filing: 22.03.2006
(51) Int. Cl.: C12N 5/071

(54) **PARATHYROID AND THYMUS TRANSPLANTATION IN DIGEORGE SYNDROME SUBJECTS**
PARATHYROID- UND THYMUSTRANSPLANTATION BEI PERSONEN MIT DI-GEORGE-SYNDROM
TRANSPLANTATION DE PARATHYROIDE ET DU THYMUS CHEZ DES SUJETS ATTEINTS PAR LE SYNDROME DE DIGEORGE

(30) Priority: 06.04.2005 US 668745 P; 19.08.2005 US 709534 P
(43) Date of publication of application: 19.12.2007
(73) Proprietor: Duke University, Durham, North Carolina 27708-0083 (US)
(72) Inventor: MARKERT, M. Louise, Durham, North Carolina 27705 (US)
(74) Representative: Chapman, Paul Gilmour
(86) International application number: PCT/US2006/010387
(87) International publication number: WO 2006/107601

(56) References cited:
- US-A- 5 968 780
- WELLS S A JR ET AL: "Parathyroid transplantation." WORLD JOURNAL OF SURGERY NOV 1977, vol. 1, no. 6, November 1977 (1977-11), pages 747-756, XP008115398 ISSN: 0364-2313
- MARKERT M LOUISE ET AL: "Thymus transplantation in complete DiGeorge syndrome: immunologic and safety evaluations in 12 patients." BLOOD 1 AUG 2003, vol. 102, no. 3, 1 August 2003 (2003-08-01), pages 1121-1130, XP002557083 ISSN: 0006-4971
- KIKUMORI T ET AL: "Parathyroid autotransplantation with total thyroidectomy for thyroid carcinoma: long-term follow-up of grafted parathyroid function." SURGERY MAY 1999, vol. 125, no. 5, May 1999 (1999-05), pages 504-508, XP005688012 ISSN: 0039-6060
- LO CHUNG-YAU: "Parathyroid autotransplantation during thyroidectomy." ANZ JOURNAL OF SURGERY DEC 2002, vol. 72, no. 12, December 2002 (2002-12), pages 902-907, XP002557084 ISSN: 1445-1433
- RICE HENRY E ET AL: "Thymic transplantation for complete DiGeorge syndrome: medical and surgical considerations." JOURNAL OF PEDIATRIC SURGERY NOV 2004, vol. 39, no. 11, November 2004 (2004-11), pages 1607-1615, XP004707879 ISSN: 1531-5037
- MARKERT ET AL: "Complete DiGeorge syndrome: Persistence of profound immunodeficiency" JOURNAL OF PEDIATRICS, MOSBY-YEAR BOOK, ST. LOUIS, MO, US, vol. 132, no. 1, 1 January 1998 (1998-01-01), pages 15-21, XP005692961 ISSN: 0022-3476
- LIAO J. ET AL.: 'Full spectrum of malformations in velo-cardio-facial syndrome/DiGeorge syndrome mouse models by altering tbx1 dosage' HUMAN MOLECULAR GENETICS vol. 13, no. 15, 2004, pages 1577 - 1585, XP003009537

## Description

### Field of the Invention

The present invention concerns the treatment of hypoparathyroidism occurring in subjects afflicted with DiGeorge syndrome.

### Background of the Invention

DiGeorge syndrome is a heterogeneous condition in which infants are born with variable deficiences of the thymus, parathyroid, and heart and often have other anomalies as well. In complete DiGeorge syndrome infants are athymic and are born with a profound T cell deficiency. DiGeorge syndrome can be treated by thymus transplantation, as described in M. Louise Markert et al., Thymus transplantation in complete DiGeorge syndrome: immunologic and safety evaluations in 12 subjects, Blood 102, 1121-1130 (2003) and M. Louise Markert et al., Transplantation of Thymus tissue in complete DiGeorge syndrome, N Eng. J. Med. 341, 1180-9 (1999).

Approximately 20% of complete DiGeorge syndrome infants have profound hypoparathyroidism. In addition, about ¾ of cases have hypoparathyroidism in general. Although thymus transplantation results in reconstitution of T cells, the ongoing hypoparathyroidism is a significant problem. The infant must take calcium replacement several times a day and is at risk of nephrocalcinosis and hypocalcemic seizures. It would be extremely useful to provide alternate ways of addressing the problem of hypoparathyroidism in DiGeorge subjects.

### Summary of the Invention

A first aspect of the present disclosure is a method of treating hypoparathyroidism in a human DiGeorge syndrome subject. The method comprises *(a)* implanting thymus tissue into the subject in an amount effective to treat the DiGeorge syndrome; and *(b)* concurrently implanting parathyroid tissue into the subject in an amount effective to treat the hypoparathyroidism.

A second aspect of the disclosure is an organ culture medium, particularly a thymus culture medium, characterized in that the organ culture medium is free of 2-deoxyguanosine.

A third aspect of the disclosure is the use of an organ culture medium, particularly a thymus culture medium, as described above, for carrying out a method as described herein.

A further aspect of the diclosure is a method of preparing live human thymus tissue for implantation into a subject in need thereof, comprising: *(a)* culturing live human thymus tissue under sterile conditions in a culture medium free of 2-deoxyguanosine for a time period sufficient to deplete mature T cells therefrom (typically at least eight days), and then *(b)* collecting the live human thymus tissue from the culture medium for implantation into a subject in need thereof (*e.g.,* a DiGeorge syndrome subject).

A further aspect of the disclosure is isolated and cultured live human thymus tissue that is both free of exogenous 2-deoxyguanosine and depleted of mature T cells (e.g., is prepared by a method as described above).

The foregoing and other objects and aspects of the present invention are explained in greater detail in the specification set forth below.

### Brief Description of the Drawings

**Figure 1** shows parathyroid hormone (PTH) levels in picograms per milliliter for the patient prior to transplantation (at day 0) and through 120 days after transplantation.
**Figure 2** shows ionized calcium levels for the patient of Figure 1 prior to, during and after transplantation, with all supplemental calcium being stopped at day 104.
**Figure 3** shows T cell function, assessed by PHA responses, for the patient of Figures 1-2, as compared to a normal adult control.
**Figure 4****.** Parathyroid hormone levels in patient DIG201 after thymus/parathyroid transplantation.
**Figure 5****.** Development of T cells in patient DIG201 after thymus parathyroid transplantation. T cells have developed with normal phenotype.

### Detailed Description of the Preferred Embodiments

"Hypoparathyroidism" as described herein is a deficiency of parathyroid hormone that causes abnormal metabolism of calcium and phosphorus. Signs of hypoparathyroidism may include one or more of low serum calcium level, elevated serum phosphorus, decreased serum parathyroid hormone level, decreased serum magnesium level, and/or abnormal heart rhythms. Symptoms of hypoparathyroidism include one or more of tingling of lips hands and feet, muscle cramps, pain in face legs or feet, abdominal pain, dry hair, brittle nails, dry scaly skin, cataracts, delayed or absent tooth formation, weakened tooth enamel; hand or foot spasms, muscle spasms or tetany, and/or convulsions or seizures.

The term "treat" as used herein refers to any type of treatment that imparts a benefit to a subject afflicted with a disease, including improvement in the condition of the subject (*e.g.,* in one or more symptoms, such as decrease the frequency and/or severity of hypocalcemic seizures), delay in the progression of the disease, etc.

"Concurrently implanting" as used herein means that the two tissues or organs are implanted at the same point in time or one immediately following the other. In the latter case, the two organs or tissues are administered at times sufficiently close that the results observed are essentially or substantially indistinguishable from those achieved when the organs or tissues are implanted at the same time.

"Depleted of mature T cells" as used herein means that the number of CD3+ T cells in the thymus has been decreased so that less than or equal to 10% of the starting number (the number found in the material to be cultured prior to culturing) remains.

**1. Thymus preparation and implantation**. Thymus tissue can be cultured and prepared for implantation in accordance with known techniques, such as described in L. Market et al., Blood 102, 1121-1130 (2003). Culturing of the tissue is carried out to deplete the thymus of mature T cells that may otherwise contribute to graft vs. host disease in the implanted subject, for example by including 2-deoxyguanosine in the culture medium. *Id.* In general such culture techniques are carried out by harvesting thymus in a sterile fashion, slicing the thymus tissue with a microtome into slices approximately 0.1 to 5 (most preferably 0.5) millimeters thick, placing the slices on a carrier or support medium or matrix (*e.g.,* filters such as MILLIPORE™ filters on surgical sponges) and in turn placing the carrier or support in organ medium in a container such as a Petri dish, still under sterile conditions, to be cultured therein. The culture media generally comprises nutrients sufficient to sustain the viability of the tissue during culturing therein, at least one antibiotic sufficient to combat microbial infection of the tissue, and a buffer system sufficient to maintain an appropriate pH range for the tissue during culture. A preferred thymus organ medium comprises or consists essentially of F12 nutrient mixture (Ham; Gibco, Grand Island, NY) with 1.36 mM 2-deoxyguanosine (Sigma, St. Louis, MO), 25 mM HEPES (*N*-2-hydroxyethylpiperazine-*N'*-2-ethanesulfonic acid; Gibco), 2 mM L-glutamine (Gibco), 10% fetal bovine serum (GIBCO), 100 ug/mL streptomycin sulfate (Gibco), 1 ug/mL gentamycin (Gibco) and 100 ug/dL Amphotericin B (BeniaSicor, Irvine, CA). The slices are maintained in a 5% CO₂ incubator at 37 °C. The medium is preferably changed daily. The tissue is cultured for at least 8, 9, 10 or 11 days, up to 3 or 4 weeks or more.

In an alternate embodiment of the invention disclosed herein, thymus tissue is collected and cultured in essentially the same manner as described above, except that the 2-deoxyguanosine is eliminated from the culture medium through the entire culturing period. An advantage of this embodiment is there is no damage to the thymic tissue from the administration of 2-deoxyguanosine and there is no residual in the cultured thymus tissue that may be transferred to the recipient. While not wishing to be bound to any particular theory of this embodiment, it is believed that depletion of mature T cells from the thymus prior to implantation is achieved by the amount of time (at least eight or ten days, up to four weeks or more, as noted above) the thymus tissue is maintained in the culture medium.

**2. Parathyroid preparation and implantation.** Parathyroid tissue need not be cultured as described above and instead may be collected from a donor and implanted directly into a subject without intervening culturing or chemical processing steps, as described in J. Olson et al., Ann. Surg. 223, 472-487 (2002) and C. Lo, ANZ J. Surg. 72, 902-907 (2002). In one embodiment the parathyroid is minced or sliced prior to implantation.

**3. Subjects and implantation.** Subjects to be treated by the methods of the present disclosure are, in general, human subjects, including both male and female subjects. In general the subjects are not more than 2 years old, and typically the subjects are not more than 18 months old.

Subjects suitable for the treatments or methods of the disclosure include both complete DiGeorge syndrome subjects who are athymic who also have profound hypoparathyroidism and complete DiGeorge syndrome subjects who have partial parathyroid deficiency as evidenced by their requiring calcium or calcitriol supplementation to prevent hypocalcemia.

Some DiGeorge syndrome subjects exhibit T cell function that poses a risk of allotransplant rejection if no immunosupression is given. Hence the subjects are preferably treated or conditioned with an immunosupressive agent prior to implantation of thymus and parathyroid to reduce the risk of transplant rejection. While any suitable immunosupressive treatment can be used, a preferred treatment is to administer anti-thymocyte antibodies (*e.g.,* rabbit anti-thymocyte globulin) to the subjects in a dose or dosages (*e.g.,* three separate doses) prior to (*e.g.,* at least one day before) the implantation, in an amount effective to combat transplant rejection of the thymus and parathyroid tissue, as described in L. Markert et al., Blood 104, 2574-2581 (2004).

Thymus tissue prepared as described herein is preferably implanted into a muscle of the subject, preferably a skeletal muscle such as quadriceps, in accordance with standard surgical procedures, preferably with the subject under local or general anesthesia. In general the amount of thymus tissue implanted is from 1 gram or 1.5 grams up to 6, 10 or 20 grams per subject (based upon the assumption that one cubic centimeter of tissue equals one gram). The thymus tissue is optionally but preferably matched to the subject for at least one of the three major HLA loci (HLA-A, HLA-B, and HLA-DR).

The parathyroid tissue is preferably implanted into a muscle of the subject, again preferably a skeletal muscle such as a quadriceps, in accordance with standard surgical procedures, preferably with the subject under local or general anesthesia. The parathyroid tissue is preferably implanted concurrently with, or during the same surgical session as, the thymus tissue (*e.g.,* when general anesthesia is employed, during the same session of general anesthesia), though it need not be, and preferably is not, implanted in the same location as the thymus tissue. The parathyroid tissue is preferably matched to the subject for at least one of the three major HLA loci (HLA-A, HLA-B, and HLA-DR), and preferably is closely matched, *e.g.,* is obtained from either a mother or father f the subject.

In general a single adult parathyroid gland is a sufficient quantity of parathyroid for implantation, although a smaller amount can be used when minced or sliced tissue is used (*e.g.,* from 20 or 40%, by weight or volume, up to the entire quantity of the parathyroid gland).

Subjects may receive cyclosporine and/or steroids in the peri transplantation period, depending upon the patient's immune status, in accordance with know techniques. Pneumocystis prophylaxis and/or intravenous immunoglobulin may be used for one or two years post transplantation, also in accordance with known techniques.

While the present invention has been described with reference to the transplantation of parathyroid tissue, it will be appreciated that other solid organs, including but not limited to heart and lung, can also be transplanted concurrently with thymus tissue instead of parathyroid tissue as described herein, to thereby reduce or inhibit rejection of the transplanted organ in an infant with complete DiGeorge syndrome.

The present invention is explained in greater detail in the following examples.

### EXAMPLE 1

### DiGeorge Syndrome Patient 1

A female infant was diagnosed with DiGeorge syndrome based on hypoparathyroidism associated with a hypocalcemic seizure on day 11, ventricular septal defects requiring surgery at 1 month, and absence of T cells noted at 5 weeks of age. The 22q11 FISH test was normal.

An unrelated postnatal thymus was obtained for transplantation. The thymus shared an HLA-DR allele with the mother and the infant. The mother was used as the parathyroid donor. Her HLA types along with those of the thymus donor and recipient are shown in the **Table** below.

| | HLA-A | HLA-B | HLA-C | HLA-DRB1 |
|---|---|---|---|---|
| Patient | **0201** | **3501** | **0401** | **0101** |
| | 0301 | 3508 | **0401** | 1401 |
| Thymus | 0101 | **3501** | **0401** | **0101** |
| | 1101 | **1801** | 0501 | 0301 |
| Parathyroid | **0201** | **3501** | **0401** | **0101** |
| | 0401 | **1801** | 0701 | 0701 |

Rabbit anti-human thymocyte globulin was used as pre-transplant conditioning. Thymus slices were inserted into the quadriceps of the recipient in an open procedure after a 2-week culture period as described in M. Markert et al., Blood 102, 1121-1130 (2003). Concurrent parathyroid transplantation was performed at the time of the thymus transplant. Donor parathyroid tissue was obtained from the mother in an open operative procedure. The parathyroid was minced into small pieces and inserted into the quadriceps at a site distinct from the thymus graft, as described in Lo et al., ANZ J. Surg. 72, 902-907 (2002). Parathyroid graft function was assessed by monitoring serum calcium and intact parathyroid hormone (PTH).

Results: There were no adverse events for the recipient or parathyroid donor relating to the transplants. The recipient has had normal PTH levels on all days post transplantation when tested. The initial test was on day 17 and the most recent test was on day 143. The patient is not on any calcium supplementation. The thymus graft biopsy at 2.5 months showed thymopoiesis (T cell development in the thymus graft). On day 157 the patient had 18% T cells with a PHA response of 205,326 counts per minute (cpm) with a background medium response of 142 cpm. This is a normal level T cell response. Remarkably on day 138, the patient had a central line infection without associated hypocalcemia or seizure. This line has been removed.

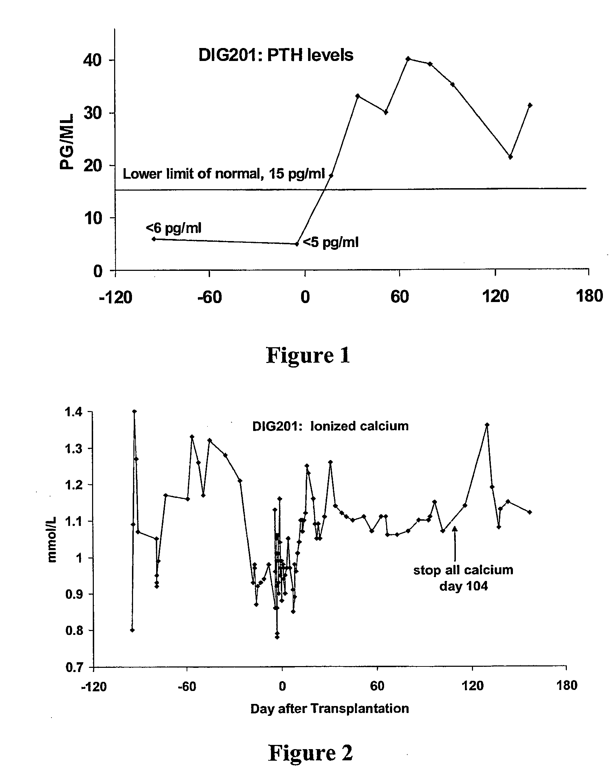

The graphs of **Figures 1-3** show the PTH and T cell function data for the patient as discussed above.

### EXAMPLE 2

### Follow-up on Patient 1 and Additional Patients

After Example 1 above, parathyroid and thymus transplantation was done in three additional infants for a total of 4 infants with complete DiGeorge syndrome (DIG201, DIG203, DIG204, and DIG206). As of this writing the patients have not had seizures from hypocalcemia after parathyroid transplantation, nor have they developed nephrocalcinosis.

There have been no adverse events associated with the parathyroid donation or the transplantation.

For patient DIG204, the parathyroid transplant was given approximately one month after thymus transplantation because the donor had a thyroid nodule that had to be biopsied prior to use of a parathyroid gland from that parent. Thus, the parental parathyroid donor had two operative procedures, one for the thyroid nodule biopsy and a second for the parathyroid organ donation. The recipient also had two operative procedures, one for thymus transplantation and one for parathyroid transplantation. The thymus was transplanted as soon as the tissue was available because T cell function is so important for the survival of the patient.

All infants have been able to come off calcium supplementation. Two patients (DIG201, DIG203) have required occasional supplementation.

DIG201 was put on calcium supplementation for approximately one week when her ionized calcium fell to 0.93 mmol/L. This has happened twice and was associated with diarrhea both times. However, without the parathyroid transplantation, it would have been necessary to keep her ionized calcium around 0.9 mmol/L to prevent nephrocalcinosis.

DIG203 was put on calcium supplementation when his calcium level fell to 7.9 mg/dl. He currently, however, has normal calcium levels without supplementation.

DIG204 has not been on calcium supplementation since her transplantation.

DIG206 was able to wean off calcium in the first two weeks after parathyroid transplantation.

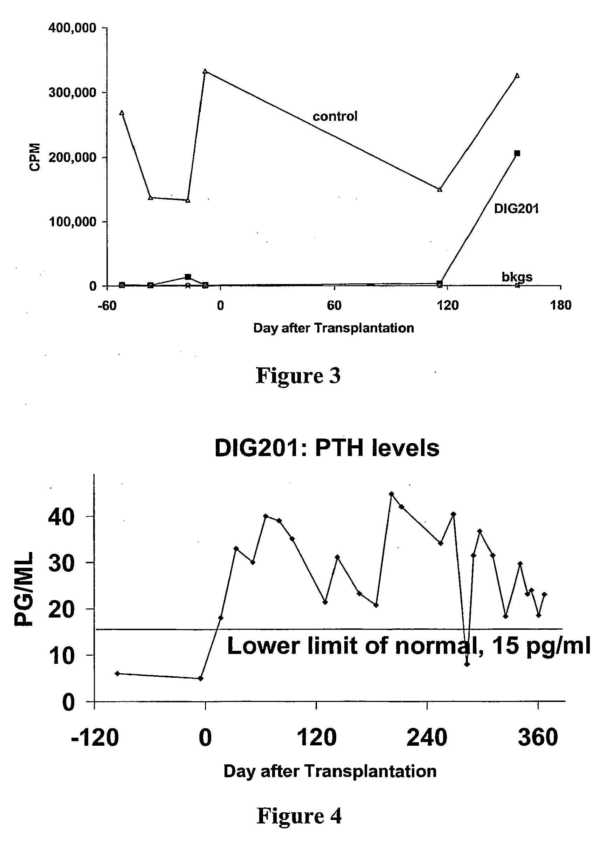

Parathyroid hormone levels have increased in all patients (the level from March 6, 2006 from the 4th patient who was transplanted February 22, 2006 is pending). See **Figure 4** for sample parathyroid hormone levels in DIG201.

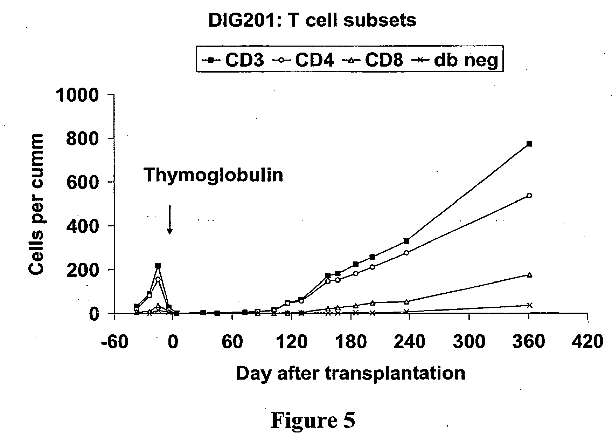

Thymic derived T cells have developed in the initial three patients (DIG201, DIG203, and DIG204). DIG206 is too close to transplantation to have developed T cells. This was a critical issue. It does not appear that the parathyroid transplant adversely affects the thymus transplant. See **Figure 5** for development of T cells in DIG201. Not shown is the normal proliferative responses of the T cells to mitogens.

**Regarding the use of thymus organ culture medium that is free of 2-deoxyguanosine**. The concern about use of culture medium free of 2-deoxyguanosine is that of graft-versus-host disease. We transplanted an infant (DIG034) using thymus cultured without 2-deoxyguanosine in the medium. The resulting thymus tissue was depleted of viable T cells and no GVHD has developed to date. The quality of the thymus tissue for transplantation is much improved without the 2-deoxyguanosine. In particular, the viability of the thymus tissue is improved in general and specifically the viability of thymic epithelium is improved.

The foregoing is illustrative of the present invention, and is not to be construed as limiting thereof. The invention is defined by the following claims.

## Claims

1. Human thymus tissue and human parathyroid tissue for use in treating hypoparathyroidism in a human complete DiGeorge syndrome subject, wherein the human thymus tissue and human parathyroid tissue are to be implanted concurrently and wherein said human thymus tissue has been cultured *in vitro* to deplete the thymus tissue of mature T cells, in a medium which is free of 2-deoxyguanosine

2. The tissue of claim, 1, wherein said thymus tissue comprises 1 to 20 grams of thymus tissue.

3. The tissue of either of claims 1 or 2, wherein said parathyroid tissue comprises at least 20% of one adult parathyroid.

4. The tissue of any preceding claim, wherein said subject is a complete DiGeorge syndrome subject with partial parathyroid function and afflicted with hypoparathyroidism.

5. The tissue of any preceding claim, wherein said subject is not more than two years old.

6. The tissue of any preceding claim, wherein the subject has been treated with anti-human thymocyte globulin prior to implanting said human thymus tissue.

7. The tissue of any preceding claim, wherein the human thymus tissue is implanted into a skeletal muscle of said subject.

8. The tissue of any preceding claim, wherein the human parathyroid tissue is implanted into a skeletal muscle of said subject.

## Patentansprüche

1. Menschliches Thymus-Gewebe und menschliches Nebenschilddrüsengewebe zur Anwendung bei der Behandlung von Hypoparathyreoidismus bei einem Subjekt mit komplettem DiGeorge-Syndrom des Menschen, wobei das menschliche Thymus-Gewebe und das menschliche Nebenschilddrüsengewebe gleichzeitig implantiert werden müssen und wobei das menschliche Thymus-Gewebe *in vitro* kultiviert wurde, um das Thymus-Gewebe von reifen T-Zellen in einem 2-Deoxyguanosin freien Medium abzureichern.

2. Gewebe nach Anspruch 1, wobei das Thymus-Gewebe 1 bis 20 Gramm Thymus-Gewebe umfasst.

3. Gewebe nach Anspruch 1 oder 2, wobei das Nebenschilddrüsengewebe mindestens 20% einer Nebenschilddrüse eines Erwachsenen umfasst.

4. Gewebe nach einem der vorangehenden Ansprüche, wobei das Subjekt ein Subjekt mit komplettem DiGeorge-Syndrom mit einer teilweisen Nebenschildrüsenfunktion ist und an Hypoparathyreoidismus leidet.

5. Gewebe nach einem der vorangehenden Ansprüche, wobei das Subjekt nicht älter als zwei Jahre alt ist.

6. Gewebe nach einem der vorangehenden Ansprüche, wobei das Subjekt vor dem Implantieren des menschlichen Thymus-Gewebes mit Anti-Human-Thymozytenglobulin behandelt wurde.

7. Gewebe nach einem der vorangehenden Ansprüche, wobei das menschliche Thymus-Gewebe in einen Skelettmuskel des Subjekts implantiert wird.

8. Gewebe nach einem der vorangehenden Ansprüche, wobei das menschliche Nebenschilddrüsengewebe in einen Skelettmuskel des Subjekts implantiert wird.

## Revendications

1. Tissu thymique humain et tissu de parathyroïde humaine pour une utilisation dans le traitement de l'hypoparathyroïdie chez un sujet atteint de syndrome de DiGeorge humain complet, le tissu thymique humain et le tissu de parathyroïde humaine devant être implantés concuremment et ledit tissu thymique humain ayant été mis en culture in vitro pour appauvrir le tissu thymique en cellules T matures, dans un milieu exempt de 2-deoxyguanosine.

2. Tissu selon la revendication 1, dans lequel ledit tissu thymique comprend de 1 à 20 grammes de tissu thymique.

3. Tissu selon la revendication 1 ou 2, dans lequel ledit tissu de parathyroïde comprend au moins 20% d'une parathyroide d'un adulte.

4. Tissu selon l'une quelconque des revendications précédentes, dans lequel ledit sujet est un sujet atteint de syndrome de DiGeorge complet avec une fonction parathyroïde partielle et souffrant de hypoparathyroïdie.

5. Tissu selon l'une quelconque des revendications précédentes, dans lequel ledit sujet est âgé de moins de deux ans.

6. Tissu selon l'une quelconque des revendications précédentes, dans lequel le sujet a été traité avec de la globuline antithymocytaire avant l'implantation dudit tissu thymique humain.

7. Tissu selon l'une quelconque des revendications précédentes, dans lequel le tissu thymique humain est implanté dans un muscle squelettique dudit sujet.

8. Tissu selon l'une quelconque des revendications précédentes, dans lequel le tissu de parathyroïde humain est implanté dans un muscle squelettique dudit sujet.
